# EUROPÄISCHE PATENTSCHRIFT

(11) **EP 1 658 093 B1**
(45) Veröffentlichungstag und Bekanntmachung des Hinweises auf die Patenterteilung: **12.09.2007**
(21) Anmeldenummer: 04704540.6
(22) Anmeldetag: 23.01.2004
(51) Int. Cl.: A61K 38/11, A61K 31/568, A61K 31/565, A61P 15/00

(54) **VERWENDUNG EINES OXYTOCIN UND TESTOSTERON ODER ESTRADIOL ENTHALTENDEN GEMISCHES ZUR BEHANDLUNG VON STÖRUNGEN DES PSYCHOSOZIALEN ERLEBENS UND VERHALTENS**
USE OF A MIXTURE CONTAINING OXYTOCIN AND TESTOSTERON OR ESTRADIOL FOR TREATING DISTURBANCES OF PSYCHOSOCIAL EXPERIENCES AND BEHAVIOUR
UTILISATION D'UN MELANGE CONTENANT DE L'OXYTOCINE ET DE LA TESTOSTERONE OU DE L'ESTRADIOL POUR TRAITER DES TROUBLES DE LA PERCEPTION ET DU COMPORTEMENT PSYCHOSOCIAL

(30) Priorität: 24.01.2003 DE 10303672
(43) Veröffentlichungstag der Anmeldung: 24.05.2006
(73) Patentinhaber: Beier, Klaus M., 14169 Berlin (DE)
(72) Erfinder: Beier, Klaus M., 14169 Berlin (DE)
(86) Internationale Anmeldenummer: PCT/DE2004/000134
(87) Internationale Veröffentlichungsnummer: WO 2004/067022

(56) Entgegenhaltungen:
- WO-A-98/43660
- US-A- 5 550 120
- US-A- 5 582 592
- DATABASE BIOSIS [Online] BIOSCIENCES INFORMATION SERVICE, PHILADELPHIA, PA, US; 1990, BLUTHE R-M ET AL: "ANDROGEN-DEPENDENT VASOPRESSINERGIC NEURONS ARE INVOLVED IN SOCIAL RECOGNITION IN RATS" XP002287533 Database accession no. PREV199090078448 & BRAIN RESEARCH, Bd. 519, Nr. 1-2, 1990, Seiten 150-157, ISSN: 0006-8993

## Beschreibung

Die Erfindung betrifft die Verwendung von pharmazeutischen Mitteln bestehend aus Oxytocin und Sexualhormonen zur Behandlung von Störungen des psychosozialen Erlebens und Verhaltens (Bindungsstörungen).

Bekannt ist, dass evolutionsbiologisch betrachtet Menschen sozial organisierte Säugetiere sind. Das bedeutet, wichtige das Erleben und Verhalten des Einzelnen stabilisierende Funktionen gehen auf soziale Bindungen zurück. Im Säuglings- und Kleinkindalter sichert die Bindung an die Eltern bzw. die entsprechenden Bezugspersonen Schutz, Zuwendung und Geborgenheit, d. h. die Erfüllung psychosozialer Grundbedürfnisse. Selbst bei ungestörter psychischer Entwicklung bleibt der Wunsch nach Bindungen, welche die genannten Grundbedürfnisse erfüllen sollen, bestehen. Dieser wird aber in der Regel mit nicht verwandten Erwachsenen und Intimpartnern verwirklicht. Bisher ist nicht hinreichend geklärt, wie das biochemische Substrat die psychische Stabilisierung durch für das jeweilige Individuum spezifische, intime Bindungen beeinflusst.

Stillende Mütter schütten durch den Stillvorgang nicht nur vermehrt Oxytocin aus, sondern werden auch in ihrem Bindungsverhalten toleranter, ruhiger und "sozialer". Dabei wird davon ausgegangen, dass der Körperkontakt zum Kind durch den Austausch von Berührungen, Wärme und Gerüchen die Ausschüttung von Oxytocin befördert (Uvnäs-Moberg, K., Psychoneuroendocrinology 23,8, S. 819-835, (1998)). Eine Korrelation zwischen der Höhe des Oxytocinspiegels und Zufriedenheit, Ausgeglichenheit sowie sozialer Kontaktfähigkeit gemessen anhand von entsprechenden Persönlichkeitstestverfahren - konnte von Nissen et al. nachgewiesen werden (Nissen, E., Gustavson, P., Widström, A. M., Uvnäs-Moberg, K., Journal of Psychosomatic Obstetrics and Gyneacology 19, S. 49-58 (1998)).

Auch konnte gezeigt werden, dass beim Menschen (Männern und Frauen) die Injektion von Oxytocin die ACTH- und Kortisolausschüttung senkt; es wird sogar der zu erwartende Anstieg von ACTH und/oder Kortisol nach der zusätzlichen Injektion von Vasopressin, mithin die "Kampf-Flucht-Reaktion" gedämpft (Chiodera, P., Salvarani, C., Bacchi-Modena, A., Spallanzani, R., Cigarini, C., Alboni, A., Gardini, E., Coiro, V., Morm. Res. 35, S. 119-123 (1991)).

Oxytocin ist eines der ersten isolierten Peptidhormone. Es ist ein Nonapeptid mit zwei Cysteinresten, die eine Disulfid-Brücke zwischen den Positionen 1 und 6 bilden und entspricht der Formel

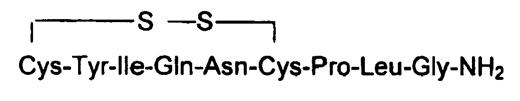

Oxytocin trägt über parasympathische Aktivierungen zur Stressreduktion und Angstlösung bei und dient dem Aufbau sozialer Bindungen (Carter, S., Annals of New York Academy of Sience 15, S. 164-174 (1997)). Der Zusammenhang von Stressverarbeitung und Oxytocinausschüttung konnte beim Menschen allerdings nicht nachgewiesen werden (Sanders, G., Freilicher, J., Lightman, S. L., Psychoneuroendocrinoloy 15, S. 47-58 (1990); Carter, S., Annals of New York Academy of Sience 15, S. 164-174 (1997)).

Die Ausschüttung des Oxytocins erfolgt pulsatil. Auch wird es relativ schnell (innerhalb von Minuten) wieder abgebaut. Aus Tierversuchen ist jedoch bekannt, dass z. B. die endogene Opioidaktivität durch Oxytocin-Injektionen steigt (Petersson, M., Alster, P., Lundberg, T., Uvnäs-Moberg, K., Neuroscience 212, S. 87-90 (1996)).

Das zeigt, dass Oxytocin über die Induktion sekundärer Neurotransmittersysteme diese länger andauernden Effekte bewirken muss.

Es gibt Befunde bei Frauen, wonach sich die Oxytocin- und Vasopressinausschüttung unter Hormonersatztherapie verändert (Bossmar, T., Forsling, M., Akerlund, M., Obstetrica et Gynecologica Scandinavica 74, S. 544-548 (1995)).

Hirn- und neurophysiologische Untersuchungen haben darüber hinaus ergeben, dass Östrogene die Genexpression für Oxytocin in bestimmten Hirnabschnitten z. B. in Abschnitten des Hypothalamus erhöhen und Östrogene sowie Progesteron die Oxytocinrezeptoren stimulieren (Pfaff, D. W.: Drive. Neurobiological and Molecular Mechanisms of Sexual Motivation,-MIT-Press, Massachusetts (1999)).

Tierversuche zeigen, dass Östrogene die Wirkung von Oxytocin verstärken (McCarthy, M. M., McDonald, C. H., Brooks, P. J., Goldman, D., Physiology & Behaviour 60, S.1209-1215 (1996)). Diese Wirkung ist auch aus der Behandlung von Schmerzsyndromen nicht organischer Ursache bekannt (Patent SE 9701161-3), wobei hier das Oxytocin an den Schmerzrezeptoren wirkt. Bisher gibt es keine Untersuchungen am Menschen über den Zusammenhang von Oxytocin/Sexualhormon und der psychosozialen Stabilisierung durch reduziertes Stresserleben und erhöhtes Wohlbefinden.

Die gesundheitserhaltende Bedeutung sozialer Unterstützung und positiver Sozialerfahrung jedoch ist gut dokumentiert. Eine gelungene partnerschaftliche Beziehung und Zugang zu sozialer Unterstützung reduzieren das Risiko für verschiedene Erkrankungen wie Herz-Kreislauf-Erkrankungen und Bluthochdruck (Ryff, C. D., Singer, B., Psychology Inquiry 9, S. 69-85 (1998)).

Bindungsstörungen können zu Beeinträchtigungen des emotionalen Erlebens, zu psychosomatischen Symptombildungen und Erkrankungen, sowie zu erheblichen Auffälligkeiten des psychosozialen Verhaltens z.B. selbst- und fremdaggressivem Beziehungs- und Kontaktverhaltens führen. Die vorgetragenen Symptome werden meist als "psychovegetative Störungen und Beschwerden", "depressive Verstimmungszustände", "Angst und/oder nervöse Unruhe" bzw. "nervöse Angst-, Spannungs- und Unruhezustände" oder auch als "emotional bedingte Unruhezustände" bezeichnet. Es werden auch Diagnosen für psychosomatische Erkrankungen wie Anorexia nervosa, Bulimie, Neurodermitis oder teilweise sogar forensisch relevante Verhaltensauffälligkeiten wie "Dissozialität" und "Delinquenz" vergeben. Es kann daher davon ausgegangen werden, dass in sehr vielen Gebieten der klinischen Medizin Patienten und Patientinnen mit unterschiedlichen Störungsbildern vorstellig werden, bei denen funktionierende und damit emotional stabilisierende soziale/intime Bindungen nicht oder nicht mehr ausreichend zur Verfügung stehen. Hierzu zählen auch chronisch erkrankte sowie ältere Menschen.

Psychisch und emotional stabilisierende Effekte ergeben sich nicht nur aus frühen sozialen Bindungen zu bedeutsamen Einzelpersonen (in der Regel der Mutter), die mit Hautkontakt, d.h. nicht-genitaler Intimität, verbunden sind, sondern auch zu Intimpartnern im Erwachsenenalter. Aus diesem Grunde ist bei der Bestimmung der optimalen Kombination von Neuropeptid (Oxytocin) und Sexualhormon die Sexualstruktur des Patienten hinsichtlich der sexuellen Orientierung auf das gleiche Geschlecht, auf das andere Geschlecht oder auf beide Geschlechter zu berücksichtigen. Diese haben zweifelsohne Auswirkungen auf die Erfüllung der biopsychosozialen Grundbedürfnisse (Nähe, Akzeptanz, Sicherheit, Geborgenheit usw.) in einer Intimbeziehung, die beispielsweise für einen auf das andere Geschlecht orientierten Menschen nur mit einem andersgeschlechtlichen Partner erreichbar sind. So kann bei gegengeschlechtlicher Orientierung das Neuropeptid (Oxytocin) auch mit einem gegengeschlechtlichen Sexualhormon kombiniert werden, z.B. bei sexuell auf Frauen orientierten Männern folglich mit einem Östrogen.

Der vorliegenden Erfindung liegt die Aufgabe zugrunde, ein pharmazeutisches Mittel zur Verfügung zu stellen, das zur psychosozialen Stabilisierung unter Berücksichtigung der individuellen Situation dient.

Die Aufgabe wurde erfindungsgemäß dadurch gelöst, dass das Mittel Oxytocin und Sexualhormone kombiniert. Es werden Kombinationen aus Oxytocin und Sexualhormonen zur Behandlung von Störungen des psychosozialen Erlebens und Verhaltens verwendet.

Bei den Sexualhormonen handelt es sich um Androgene und/oder Östrogene oder deren physiologisch verträglichen Salze oder Ester. Vorzugsweise handelt es sich bei den Sexualhormonen um Testosteron bzw. Dihydrotestosteron und/oder Estron und/oder Estriol und/oder Estradiol und/oder Ethinylestradiol.

Das Oxytocin wird in einer Menge von 1 µg bis 200 µg pro kg Körpergewicht des Patienten als Tagesdosis verabreicht. Die Sexualhormone werden in Mengen von 0,05 µg bis 200 µg, pro kg Körpergewicht als Tagesdosis verabreicht.

### Beispiel 1: Untersuchungen zum Einfluss der Kombination Oxytocin/ Sexualhormon auf den psychophysiologischen Zustand von Probanden.

Untersucht wurden 4 Probanden, zwei heterosexuelle Paare mit jeweils mehr als 20jähriger Partnerschaftsdauer. In einer Doppelblindstudie wurden Oxytocin gegen Placebo, die Kombinationen Oxytocin/Estradiol an Männern und Oxytocin/Testosteron an Frauen gegen Placebo und gegen den "Austausch von Intimität" untersucht.

Den Männern wurde Oxytocin nasal als Spraystoß (1 Spraystoß Syntocinon® = 4 I.E. Oxytocin) und Estradiol dermal als Membranpflaster(2 mg Estradiol mit der Freisetzungsrate von ca. 0,03 mg/h; Estraderm TTS® 25) verabreicht.

Den Frauen wurde Oxytocin nasal als Spraystoß (1 Spraystoß Syntocinon® = 4 I.E. Oxytocin) und Testosteron dermal als Membranpflaster (12,2 mg Testosteron mit einer Freisetzungsrate von ca. 0,1 mg/h; Androderm® 2,5 mg) verabreicht.

Die Verabreichung von Oxytocin erfolgte nasal und doppelblind (Placebo und Verum) in zwei Dosierungen, 1 Sprühstoß = 4 I.E. Oxytocin und zwei Sprühstöße = 8 I.E. Oxytocin, jeweils fortlaufend im Abstand von einer Stunde. Oxytocin wurde sowohl vor als auch nach der Anlage des Membranpflasters verabreicht. Insgesamt wurden 4 nasale Interventionen vor und nach der zusätzlichen Sexualhormongabe durchgeführt, wobei das Verum 2 mal in unterschiedlichen Dosierungen gegeben wurde. Die ersten nasalen Interventionen erfolgten nach 2 Stunden Wirkzeit der Sexualhormone.

Ausgewertet wurden Messungen ab 1 Stunde nach Aufkleben des Pflasters im hinteren Hüftbereich.

Es wurden psychophysiologische Reaktionsmuster anhand der Parameter Herzfrequenz, Pulsfrequenz, EMG, Hautpotential, Hautwiderstand und Hauttemperatur durch SMARD-Watch gemessen und subjektive Zustandsbeschreibungen über einen Fragebogen bewertet.

Die Pulsfrequenzdaten wurden über ein Zeitintervall von 10 Sekunden gemittelt. Die Daten für Hautwiderstand, Hautpotential und EMG wurden mit einer Abtastrate von einer Sekunde erfasst. Zur weiteren Datenanalyse wurden diese über ein Zeitintervall von 10 Sekunden gemittelt.

Für die Auswertung wurde eine sogenannte Dynamikanalyse angewandt. Hierfür wurden anhand der protokollarischen Aufzeichnungen der Auswertebereich für die einzelnen Applikationen wie z.B. der Einsatz des Verum-Nasensprays definiert. Die kontinuierlich gemessenen Daten wurden dann in diesen Auswertungsfenstern auf besondere Veränderungen durchgesehen, z.B. eine Verringerung des Hautwiderstandes u.ä., um die physiologischen Korrelate des emotionalen Geschehens zu bestimmen.

Bei der visuellen Auswertung der Dynamikanalysen waren zum Zeitpunkt der Applikationen in der Regel zwei Punkte auffällig:
1. eine relativ starre Regulation zum Zeitpunkt der Applikation (Anspannung; stärkere Sympathikusaktivität),
2. eine mit einer Verzögerung von 3 bis 4 Minuten auftretende Verlangsamung der Regulationsvorgänge (Entspannung; gegenläufige, parasympathische Aktivierung).

Die mathematische Auswertung erfolgte über die Bestimmung von Kreuzkorrelationen als Maß für die Bestimmung des Zusammenhanges zwischen zwei Körperfunktionen. Da die Daten der verschiedenen Funktionen zum gleichen Zeitpunkt gemessen wurden, lagen zur Auswertung jeweils Zeitreihen vor, beispielsweise Daten für EMG und Hautwiderstand, die in 1-Sekunden-Intervallen über die Zeit von 100 Sekunden gemessen wurden. Von diesen Originaldaten wurde die Kovarianz oder der Kreuzkorrelationskoeffizient berechnet, der die Ähnlichkeit zwischen den beiden Datensätzen beschreibt. Nimmt man zum Beispiel zwei Sinusfunktionen von gleicher Amplitude, Phase und Frequenz, so ergibt die Korrelation den Wert 1. Sind Amplitude und Frequenz gleich, die Phasen aber um 180 Grad verschoben, ergibt der Korrelationswert den Wert -1. Bei unterschiedlichen Funktionen ergeben sich zwangsläufig Werte zwischen -1 und +1. Mathematisch wird das durch punktweises Aufsummieren von Differenzen zwischen je zwei Messpunkten der beiden Zeitreihen bei verschiedenen Verschiebungen zueinander gelöst.

Um nun festzustellen, ob und wie sich die Beziehung zwischen zwei Körperfunktionen über die Zeit ändert, wurden anhand längerer Messabschnitte die Korrelationswerte Punkt für Punkt berechnet. Diese "dynamische Kreuzkorrelation" stellt die Dynamik der Beziehung zweier Funktionen dar, indem es Auskunft darüber gibt, wann und wie stark sich ihr Korrelationswert ändert.

Tabelle 1 zeigt die über alle Probanden gemittelten Kreuzkorrelationskoeffizienten für die einzelnen Applikationen.

**Tabelle 1: Gemittelte Kreuzkorrelationskoeffizienten für alle Applikationen**

| **Art der Applikation** | **Kreuzkorrelationskoeffizient** |
|---|---|
| Austausch von Intimität | 0,45 |
| Placebo | 0,58 |
| Oxytocin | 0,51 |
| Placebo/Sexualhormon | 0,37 |
| Oxytocin/Sexualhormon | 0,25 |

Die gemittelten Werte der Kreuzkorrelationskoeffizienten ergaben für die Oxytocinapplikation einen niedrigeren Wert als für die Gabe des Placebos, bei der Applikation von Oxytocin/Sexualhormon erhält man einen noch kleineren Wert. Niedrige Korrelationskoeffizienten sind hierbei ein Indiz dafür, dass sich sympathische und parasympathische Regulationsvorgänge gegenseitig ausgleichen, während hohe Koeffizienten ein Ungleichgewicht zum Ausdruck bringen.

Überraschenderweise lag der Korrelationskoeffizient nach Gabe von Oxytocin/Sexualhormon noch niedriger als nach der Applikation von "Austausch von Intimität".

Die Auswertung der subjektiven Zustandsbeschreibungen ergab nach Gaben von Oxytocin und von Oxytocin/Sexualhormon, dass die Selbstwahrnehmung stärker in Richtung Entspannung tendierte.

### Beispiel 2:

In der klinischen Anwendung konnte bei zwei Patienten mit unterschiedlich stark ausgeprägten Störungen des psychosozialen Erlebens und Verhaltens (Bindungsstörungen) folgendes beobachtet werden:
1. Beobachtung bei stark ausgeprägter Symptomatik: Der Patient ist ein 43jähriger Mann mit hirnorganischen Psychosyndrom bei Zustand nach Aneurysma-Hirnblutung im Jugendalter. Es besteht eine deutliche Beeinträchtigung der psychosozialen Integration und bisher lebenslange Partnerlosigkeit mit hochgradiger Lebensunzufriedenheit. Zwei Stunden nachdem ein Membranpflaster (2 mg Estradiol mit der Freisetzungsrate von ca. 0,03 mg/h; Estraderm TTS® 25) angelegt worden war, wurde in Stundenabständen Oxytocin als Spraystoß nasal (2 mal 4 I.E., 2 mal 8 I.E. Syntocinon®-Spray) verabreicht. Dies führte zu einer deutlichen Verbesserung der Grundbefindlichkeit. Auf einer Skala von 0 (ganz schlecht) bis 10 (sehr gut) plazierte sich der Patient vor der Anwendung zwischen 1 und 2, am Ende zwischen 6 und 7. Dies waren Werte, die er sonst selten erreicht. Der Effekt hielt den nächsten Tag über an.
2. Beobachtungen bei mäßig ausgeprägter Symptomatik: Ein 57jähriger Mann in einer Belastungssituation mit deutlichen sozialen und partnerschaftlichen Auswirkungen im Sinne des Rückzuges und der reduzierten Zuwendung berichtet nach Anwendung der Kombination von Estradiolvalerat (2 mg oral) und Oxytocin (8 I.E. nasal) eine psychosoziale Stabilisierung mit erhöhter kommunikativer Zuwendungsbereitschaft zur Partnerin.

## Patentansprüche

1. Verwendung einer Oxytocin und die Sexualhormone Testosteron oder Estradiol oder deren Derivate oder physiologisch verträglichen Salze oder Ester enthaltenden Wirkstoff-Kombination zur Herstellung eines Medikamentes zur Behandlung von Störungen des psychosozialen Erlebens und Verhaltens (Bindungsstörungen).

2. Verwendung einer Wirkstoff-Kombination zur Herstellung eines Medikamentes nach Anspruch 1 **dadurch gekennzeichnet, dass** das Oxytocin in einer Menge von 20 µg bis 200 µg pro kg Körpergewicht des Patienten als Tagesdosis eingesetzt wird.

3. Verwendung einer Wirkstoff-Kombination zur Herstellung eines Medikamentes nach Anspruch 1 bis 2 **dadurch gekennzeichnet, dass** das Sexualhormon in einer Menge von 0,05 µg bis 200 µg pro kg Körpergewicht des Patienten, als Tagesdosis eingesetzt wird.

## Claims

1. Use of an active substance combination containing oxytocin and the sexual hormones testosterone or estradiol or their derivates or physiologically tolerated salts or esters for production of a medicine to treat disorders of psychosocial experience and behaviour (bonding disorders).

2. Use of an active substance combination for the production of a medicine in compliance with the claims of 1.) determined by a treatment using a daily dose ranging from 20 µg of oxytocin to 200 µg per kilogram body weight of the patient.

3. Use of an active substance combination for the production of a medicine in compliance with 1.) and 2.) determined by a treatment using a daily dose of the sexual hormone ranging from 0,05 µg to 200 µg per kilogram body weight of the patient.

## Revendications

1. L'emploi d'une composition de substances contenant de l'oxytocine et des hormones sexuelles testosterone ou estradiole ou de leurs dérivés ou des sels ou esters tolérés par l'organisme, pour la production d'un médicament traitant les troubles de la perception et comportement psychosociaux (troubles relationnels)

2. L'emploi d'une composition de substances pour la production d'un médicament selon "1" indiquant le dosage d'oxytocine de 20 µg à 200 µg par kg du poids du patient par jour administré à celui-ci.

3. L'emploi d'une composition de substances pour la production d'un médicament selon "1" à "2" indiquant le dosage de l'hormone sexuelle de 0,05 µg à 200 µg par kg du poids du patient par jour administré à celui-ci.
